# EUROPEAN PATENT APPLICATION

(11) **EP 4 344 659 A1**
(43) Date of publication of application: **03.04.2024**
(21) Application number: 22901269.5
(22) Date of filing: 29.11.2022
(51) Int. Cl.: A61B 17/29

(54) **FORCEPS DEVICE**

(30) Priority: 30.11.2021 JP 2021193713
(71) Applicant: Public University Corporation Yokohama City University, Yokohama-shi, Kanagawa 236-0027 (JP); Nara Seiko Inc., Sakurai-shi, Nara 633-0101 (JP)
(72) Inventor: NAKAYAMA, Meijin, Yokohama-shi, Kanagawa 236-0027 (JP); ORIDATE, Nobuhiko, Yokohama-shi, Kanagawa 236-0027 (JP); NAKAGAWA, Hiroo, Sakurai-shi, Nara 633-0101 (JP); NISHIURA, Sunao, Sakurai-shi, Nara 633-0101 (JP); KITAMURA, Hitomi, Sakurai-shi, Nara 633-0101 (JP)
(74) Representative: Manitz Finsterwald Patent- und Rechtsanwaltspartnerschaft mbB
(86) International application number: PCT/JP2022/043868
(87) International publication number: WO 2023/100837

(57) **Abstract**

Provided is a forceps device (1) configured to easily change a state of a ratchet mechanism (19). The forceps device (1) includes an opening/closing drive unit (6) that opens an opening/closing portion (4) when positioned at an open position (O) and closes the opening/closing portion (4) when positioned at a closed position (C), and a ratchet mechanism (19) transitions between a restriction state in which the opening/closing drive unit (6) is restricted to move in the open position (O) direction and a restriction release state in which the move is not restricted. The ratchet mechanism (19) includes an operation portion (27) that projects rearward from a main body (3) of the forceps device (1) and is operated to cause the transition of the state of the ratchet mechanism (19) .

## Description

### TECHNICAL FIELD

The present invention relates to a forceps device.

### BACKGROUND ART

Conventionally, there has been known a forceps device provided with a fixed handle fixed to a device main body and a movable handle turnably disposed to the device main body. The forceps device is configured to open and close an opening/closing portion at a device distal end coupled to the movable handle by turning the movable handle (for example, see Patent Document 1). The forceps device of Patent Document 1 includes a ratchet mechanism configured to allow the movable handle to be turned in the direction of the fixed handle and inhibit the turning in the opposite direction in a predetermined state.

The ratchet mechanism includes a ratchet with a pawl turnably supported by the main body, and ratchet teeth provided to the movable handle. The ratchet is biased in a direction in which the pawl is engaged with the ratchet teeth, and operated by a ratchet release switch.

The ratchet release switch is held by the main body freely displaceable between a first position at which the turn of the ratchet is not interfered and a second position at which a cam portion presses the ratchet to forcibly separate the pawl from the ratchet teeth.

An operation to displace the ratchet release switch from the first position to the second position is performed by inserting a finger behind an operation portion of the ratchet release switch and pushing the operation portion forward. An operation to change the position of the ratchet release switch from the second position to the first position is performed by putting the finger ahead of the operation portion and pulling the operation portion rearward.

Then, in a state where the ratchet release switch is positioned at the first position, by bringing the movable handle close to the fixed handle, the opening/closing portion of a forceps device function unit can be closed, and meshing of the pawl of the ratchet with the ratchet teeth allows keeping the state. On the other hand, in a state where the ratchet release switch is positioned at the second position, by spacing the movable handle from the fixed handle, the opening/closing portion of the forceps device function unit can be opened.

Patent Document 1: JP-A-2002-11019

### DISCLOSURE OF THE INVENTION

### PROBLEMS TO BE SOLVED BY THE INVENTION

However, according to the forceps device of Patent Document 1, to mutually switching the states of the ratchet mechanism between the state where the ratchet release switch is positioned at the first position and the state where the ratchet release switch is positioned at the second position, it is necessary to alternately perform the operation of pushing the operation portion forward with the finger inserted behind the operation portion of the ratchet release switch and the operation of pulling the operation portion rearward with the finger put ahead of the operation portion. Therefore, the operation to switch the state of the ratchet mechanism is complicated.

In consideration of the problem of the conventional technique, it is an object of the present invention to provide a forceps device with a ratchet mechanism whose states are easily switchable.

### SOLUTIONS TO THE PROBLEMS

A forceps device of the present invention includes:
a main body with a fixed handle extending downward;
an opening/closing function unit having a base end portion supported by the main body and a distal end portion, the opening/closing function unit extending forward from the main body and including an opening/closing portion to be opened and closed at the distal end portion;
an opening/closing drive unit serially coupled to a rear end portion of the opening/closing function unit and supported by the main body to be movable between an open position in a front side and a closed position in a rear side, the opening/closing drive unit opening the opening/closing portion when positioned at the open position and closing the opening/closing portion when positioned at the closed position;
a movable handle supported by the main body to be movable between a forward position spaced forward from the fixed handle and a rearward position close to the fixed handle, the movable handle being coordinated with the opening/closing drive unit such that the opening/closing drive unit is positioned at the open position when the movable handle is positioned at the forward position and the opening/closing drive unit is positioned at the closed position when the movable handle is positioned at the rearward position; and
a ratchet mechanism that transitions between a restriction state in which the opening/closing drive unit is restricted to move in a direction of the open position and allowed to move in a direction of the closed position and a restriction release state in which the move in the open position direction and the closed position direction is not restricted, wherein
the ratchet mechanism includes:
   ratchet teeth including a plurality of teeth arranged in a front-rear direction at a rear end portion of the opening/closing drive unit; and
   a stopper supported by the main body to be turnable around a turning axis line extending in a left-right direction between a first turning position at which the ratchet mechanism is transitioned to the restriction state and a second turning position at which the ratchet mechanism is transitioned to the restriction release state,
the stopper includes:
   a ratchet pawl disposed in a distal end side with respect to the turning axis line of the stopper, the ratchet pawl being configured to allow the opening/closing drive unit to move rearward without meshing with the ratchet teeth and restrict the opening/closing drive unit to move forward by meshing with the ratchet teeth when the stopper is positioned at the first turning position; and
   an operation portion disposed in a rear end side with respect to the turning axis line of the stopper so as to project rearward from the main body, the operation portion being operated to turn the stopper between the first turning position and the second turning position,
first biasing means that biases the stopper from the second turning position toward the first turning position is disposed between the main body and the stopper, and
opening/closing drive unit biasing means that biases the opening/closing drive unit in a direction from the closed position toward the open position is disposed between the main body and the stopper.

In the present invention, when the operation portion of the stopper is operated against the biasing force of the first biasing means and the stopper is positioned at the second turning position, the ratchet mechanism is in the restriction release state in which the restriction on the move of the opening/closing drive unit in the open position direction and the closed position direction are released. In this state, when the movable handle is loosened, since the opening/closing drive unit is positioned at the open position by the opening/closing drive unit biasing means, the opening/closing portion is opened, and the movable handle is coordinated with the opening/closing drive unit to be positioned at the forward position.

In this state, when the movable handle is turned to the rearward position against the biasing force of the opening/closing drive unit biasing means, the opening/closing drive unit is coordinated to this to be moved to the closed position, and the opening/closing portion is closed. Therefore, by gripping the fixed handle and the movable handle with one hand and adjusting the grip strength, the opening/closing portion can be freely opened and closed.

Meanwhile, when the operation of the operation portion of the stopper is loosened and the stopper is positioned at the first turning position according to the biasing force of the first biasing means, the ratchet mechanism transitions to the restriction state in which the move of the opening/closing drive unit in the open position direction is restricted. In this state, while the move of the opening/closing drive unit to the closed position in the rear side is allowed, the move to the open position in the front side is restricted by the ratchet pawl meshing with the ratchet teeth.

Therefore, by gripping the fixed handle and the movable handle with one hand and turning the movable handle to the rearward position, the opening/closing drive unit can be moved from the open position to the closed position to close the opening/closing portion. In this state, even when the movable handle is loosened, since the move of the opening/closing drive unit to the open position is restricted, the closed state of the opening/closing portion is maintained.

Accordingly, the present invention allows the opening/closing portion to be opened and closed while the ratchet mechanism is switched between the restriction release state and the restriction state by the operation portion of the stopper at the appropriate timing. The operation portion is disposed in the rear end side with respect to the turning axis line of the stopper so as to project rearward from the main body of the forceps device. Therefore, the ratchet mechanism can be easily switched by operating the operation portion with the thumb while performing the open/close operation with one hand and gripping the fixed handle and the movable handle.

The present invention is preferably provided with fixing means that fixes the stopper at the first turning position or the second turning position to be releasable by the operation portion when the stopper is turned to the first turning position or the second turning position.

Accordingly, since the stopper can be fixed at the first turning position and the second turning position to be releasable by the operation of the operation portion, the operation of the movable handle in the state where the stopper is positioned at the second turning position can be easily performed with the thumb released from the operation portion.

In this case, the fixing means may include:
a ball plunger disposed at one of the main body or the stopper; and
a first recessed portion and a second recessed portion formed at the other of the main body or the stopper, the first recessed portion and the second recessed portion functioning as receiving sides of the ball plunger when the stopper is positioned at the first turning position and the second turning position, and
the first recessed portion may have a length in a turning direction enough to allow the stopper to slightly turn between a turning position at which the ratchet pawl meshes with the ratchet teeth and a turning position at which the meshing is released when the stopper is positioned at the first turning position.

Accordingly, in the restriction state in which the ball plunger is positioned at the first recessed portion, the ratchet is allowed to slightly turn between the turning position at which the ratchet pawl meshes with the ratchet teeth and the turning position at which the meshing is released. Therefore, the function of the ratchet mechanism to restrict the move of the opening/closing drive unit in the open position direction and allow the move in the closed position direction in the restriction state can be ensured.

Even when the stopper is positioned at the first turning position to make the ratchet mechanism in the restriction state, by slightly operating the operation portion of the stopper by a small amount with the thumb, the movable handle can be operated while the ratchet mechanism is freely transitioned between the meshing state of the ratchet pawl with the ratchet teeth and the meshing release state.

In the present invention, the opening/closing function unit may include:
an outer pipe portion supported by the main body to be rotatable with a knob, the outer pipe portion supporting the opening/closing portion to be openable and closable at the distal end portion;
a drive shaft disposed in the outer pipe portion and driven in the front-rear direction to open and close the opening/closing portion; and
coupling means that serially couples the drive shaft to the opening/closing drive unit to be releasable.

Accordingly, by the move of the opening/closing drive unit between the open position and the closed position, the drive shaft can be driven in the front-rear direction via the coupling means, thereby opening and closing the opening/closing portion. Additionally, by releasing the coupling of the drive shaft to the opening/closing drive unit by the coupling means to remove the opening/closing function unit from the main body, and attaching another opening/closing function unit to the main body and coupling its drive shaft to the opening/closing drive unit, the opening/closing function unit can be easily replaced.

In the present invention, the opening/closing function unit may include a shaft supporting portion that supports the drive shaft to be slidable in the front-rear direction,
the main body may have a front end portion provided with an insertion fixing portion into which a rear end side of the shaft supporting portion is inserted to be fixed,
the shaft supporting portion may include left and right press buttons in a front end side,
each of the left and right press buttons may be supported to be movable in the left-right direction while being biased in an opposite direction of the drive shaft by button biasing means at the shaft supporting portion,
the drive shaft may be provided with a contracted part having a tapered surface with a diameter increasing toward the rear side,
each of the press buttons may be provided with a pressing portion in the drive shaft side, the pressing portion presses the tapered surface to slide the drive shaft to the rear side, and
the coupling means may couple the drive shaft to the opening/closing drive unit based on the slide of the drive shaft to the rear side.

Accordingly, by fixing the shaft supporting portion of the opening/closing function unit to the front end portion of the main body, and then pressing the left and right press buttons of the shaft supporting portion, the drive shaft can be coupled to the opening/closing drive unit.

In this case, the drive shaft may have a rear end portion provided with an enlarged-diameter portion having a tapered surface with a diameter increasing toward the front side until the diameter becomes larger than a diameter of the drive shaft,
the coupling means may include a shaft holder with a pressing portion,
the shaft holder may be held by the opening/closing drive unit to be movable in the left-right direction between a projection position at which the pressing portion projects from the main body and a push-in position at which the pressing portion is pushed into the main body, and the shaft holder may be biased in a direction from the push-in position to the projection position by the holder biasing means,
the shaft holder may be provided with an engaging hole that releasably captures the enlarged-diameter portion of the drive shaft,
the engaging hole may be configured of:
   a large-diameter portion having a diameter that allows the pressing portion the enlarged-diameter portion to pass through the large-diameter portion; and
   a U-shaped portion located in the push-in position side of the shaft holder with respect to the large-diameter portion, the U-shaped portion having a width smaller than a largest diameter of the enlarged-diameter portion and larger than a diameter of the drive shaft, the U-shaped portion being coupled to the large-diameter portion, and
a taper-shaped portion may be provided in the front side of the U-shaped portion of the shaft holder, the taper-shaped portion may allow the enlarged-diameter portion to pass through the large-diameter portion by contacting the tapered surface of the enlarged-diameter portion to move the shaft holder to the push-in position against the biasing force of the holder biasing means when the drive shaft slides rearward.

Accordingly, when the shaft supporting portion of the opening/closing function unit is fixed to the front end portion of the main body, and then the left and right press buttons of the shaft supporting portion are pressed, the drive shaft slides rearward. Therefore, the tapered surface of the enlarged-diameter portion of the drive shaft contacts the taper-shaped portion of the shaft holder and moves the shaft holder to the push-in position, and thus the enlarged-diameter portion passes through the large-diameter portion of the shaft holder. Corresponding to this, since the holder biasing means returns the shaft holder to the projection position, the U-shaped portion of the engaging hole of the shaft holder captures the enlarged-diameter portion of the drive shaft. Then, the serial coupling of the drive shaft and the opening/closing drive unit is completed. Accordingly, the coupling of the drive shaft and the opening/closing drive unit can be reliably achieved with the simple configuration.

The shaft supporting portion may have an outer surface in a rear end side thereof, the shaft supporting portion being provided with an engaging recess at the outer surface,
the front end portion of the main body may be provided with a through hole and an engaging pin, the through hole may be formed from an outer surface of the front end portion to an inner surface of the insertion fixing portion, and the engaging pin may be disposed in the through hole,
the main body may have an outer surface provided with a leaf spring, and the leaf spring biases the engaging pin from an outside toward an inside of the through hole, and
the shaft supporting portion may be configured to be fixed to the insertion fixing portion by fitting of the engaging pin to the engaging recess due to a biasing force of the leaf spring when the shaft supporting portion is inserted into the insertion fixing portion.

Accordingly, by inserting the shaft supporting portion into the insertion fixing portion of the main body, the engaging pin of the through hole is fitted to the engaging recess of the shaft supporting portion, and the shaft supporting portion is fixed to the insertion fixing portion. Therefore, the shaft supporting portion can be reliably fixed to the main body.

In the present invention, the opening/closing drive unit biasing means may include:
second biasing means disposed between the main body and the opening/closing drive unit, the second biasing means biasing the opening/closing drive unit in a direction from the closed position toward the open position; and
third biasing means disposed between the main body and the movable handle, the third biasing means biasing the movable handle in a direction separating from the movable handle to the frond side.

Accordingly, since the second biasing means that biases the opening/closing drive unit in the direction from the closed position toward the open position can be assisted by the third biasing means, the second biasing means can be compactly configured.

In the present invention, the movable handle may be supported by the main body to be turnable around a spindle that is located above the opening/closing drive unit and extends in the left-right direction, and
the opening/closing drive unit may be coordinated with the movable handle via an oblong hole that is provided below the spindle at the movable handle and extends in an up-down direction and a pin-shaped member that is provided to the opening/closing drive unit and passes through the oblong hole in the left-right direction.

Accordingly, the opening/closing drive unit can be coordinated with the movable handle while the large move of the movable handle is converted to the small move of the opening/closing drive unit. This allows the appropriate arrangement of respective units of the forceps device while achieving the comfortable operation of the movable handle.

In the present invention, the outer pipe portion may include an opening/closing portion holder that has a distal end and holds the opening/closing portion at the distal end,
the opening/closing portion holder may include a drive unit housing portion having a slit shape with an open distal end side and having both sidewalls,
the opening/closing portion may include:
   two blade portions;
   a blade turning shaft that turnably couples rear end sides of the two blade portions, the blade turning shaft having both end portions being supported by both of the sidewalls of the drive unit housing portion; and
   two link members having respective distal end portions turnably coupled to the rear end portions of the blade portions, the rear end portions being coupled to one another and turnably coupled to a distal end portion of the drive shaft,
the two blade portions may be curved in an identical direction with respect to a turning plane perpendicular to the blade turning shaft in a closed state, and may function as scissors to be opened and closed by driving of the drive shaft in the front-rear direction, and
the blade turning shaft may be configured of a bolt fastened to the drive unit housing portion with a nut.

Accordingly, the forceps device of the present invention can be used as scissors forceps.

### BRIEF DESCRIPTION OF THE DRAWINGS

FIG. 1 is a perspective view of a forceps device according to a first embodiment of the present invention in a state where a part of a case is removed.
FIG. 2 is a cross-sectional view of illustrating a main part of the forceps device of FIG. 1.
FIG. 3 is a drawing illustrating a ratchet mechanism of the main part of FIG. 2.
FIG. 4A to FIG. 4C are cross-sectional views illustrating respective states that a ball plunger of the forceps device of FIG. 1 can have.
FIG. 5A to FIG. 5D are drawings illustrating a function of the ratchet mechanism.
FIG. 6A is a drawing of a shaft holder of the forceps device of FIG. 1 viewed from front, and FIG. 6B is a drawing of the shaft holder viewed from back.
FIG. 7A to FIG. 7C are cross-sectional views illustrating an operation of coupling means in the forceps device of FIG. 1.
FIG. 8A and FIG. 8B are cross-sectional views illustrating an operation of a shaft supporting portion when an opening/closing function unit of the forceps device of FIG. 1 is attached.
FIG. 9 is a drawing illustrating a state where a left case of a forceps device according to a second embodiment of the present invention is removed viewed from left side.
FIG. 10A is a cross-sectional view illustrating a state of surely attaching an opening/closing function unit of the forceps device of FIG. 9 to a main body.
FIG. 10B is another cross-sectional view illustrating a state of surely attaching the opening/closing function unit of the forceps device of FIG. 9 to the main body.
FIG. 11 is a perspective view illustrating a distal end portion of the opening/closing function unit of the forceps device of FIG. 9.
FIG. 12 is a cross-sectional view of the opening/closing function unit of FIG. 11 in a closed state taken along a surface passing through a center axis line of a blade turning shaft and distal ends of respective blade portions.

### DESCRIPTION OF PREFERRED EMBODIMENTS

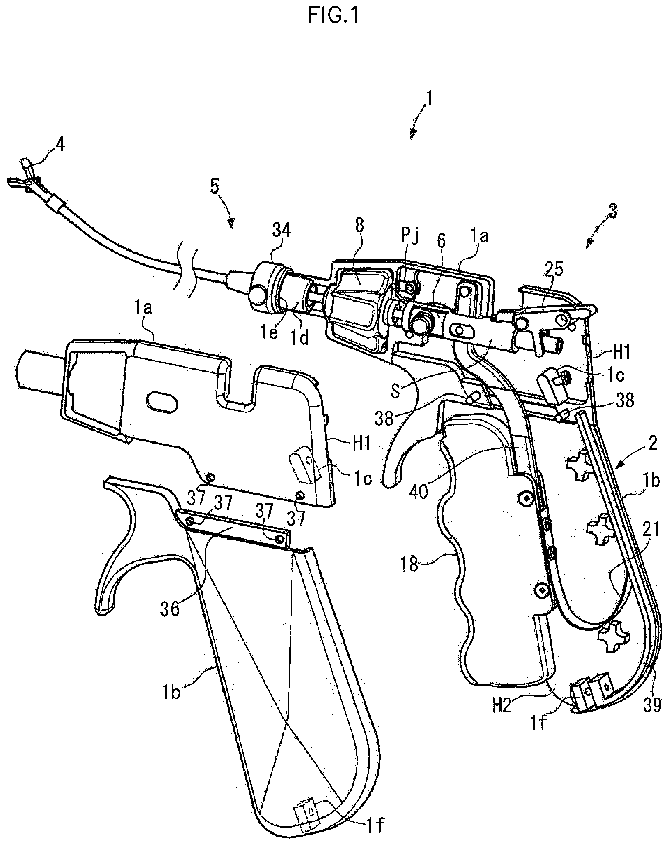

The following describes embodiments of the present invention with reference to the drawings. FIG. 1 illustrates a forceps device according to a first embodiment of the present invention. As illustrated in the drawing, a forceps device 1 includes a main body 3 with a fixed handle 2 extending downward, and an opening/closing function unit 5 having a base end portion supported by the main body 3, extending forward from a front end of the main body 3, and including an opening/closing portion 4 operated to be opened and closed at a distal end portion. The opening/closing function unit 5 is supported at a distal end portion of the main body 3 via a shaft supporting portion 34.

The main body 3 is configured with right and left upper cases 1a and right and left lower cases 1b. The left upper case 1a and the left lower case 1b are mutually coupled by fitting a fitting portion 36, which is provided at an upper end edge of the left lower case 1b and extends in a front-rear direction, into a groove, which is provided at a lower end edge of the left upper case 1a and extends in the front-rear direction, and inserting pins 38 through holes 37 provided at both cases to fix them to one another, thus constituting a left side case.

FIG. 1 illustrates a state where the left upper case 1a and the left lower case 1b are removed. The right upper case 1a and the right lower case 1b are similarly coupled, thus constituting a right side case.

The right and left upper cases 1a are fixed to one another by screwing upright pieces 1c disposed upright on insides of the respective right and left upper cases 1a to one another and fitting semi-cylindrical distal end portions of the right and left upper cases 1a into an annular groove portion 1e of a cylindrical insertion fixing portion 1d externally fitted to a rear side of the shaft supporting portion 34. The screwing is performed through an access hole H1 formed by end edges in the rear side of the right and left upper cases 1a.

The right and left lower cases 1b are fixed to on another by screwing upright pieces 1f disposed upright on insides of the respective right and left lower cases 1b to one another, thus constituting a housing of the fixed handle 2. The screwing is performed through an access hole H2 formed by end edges in the lower side of the right and left lower cases 1b. From the inside of the rear side end edge to the inside of the lower side end edge of the right and left lower cases 1b, a reinforcement member 39 is disposed. A lower end portion of the reinforcement member 39 may be screwed to them together with the upright pieces 1f of the right and left lower cases 1b.

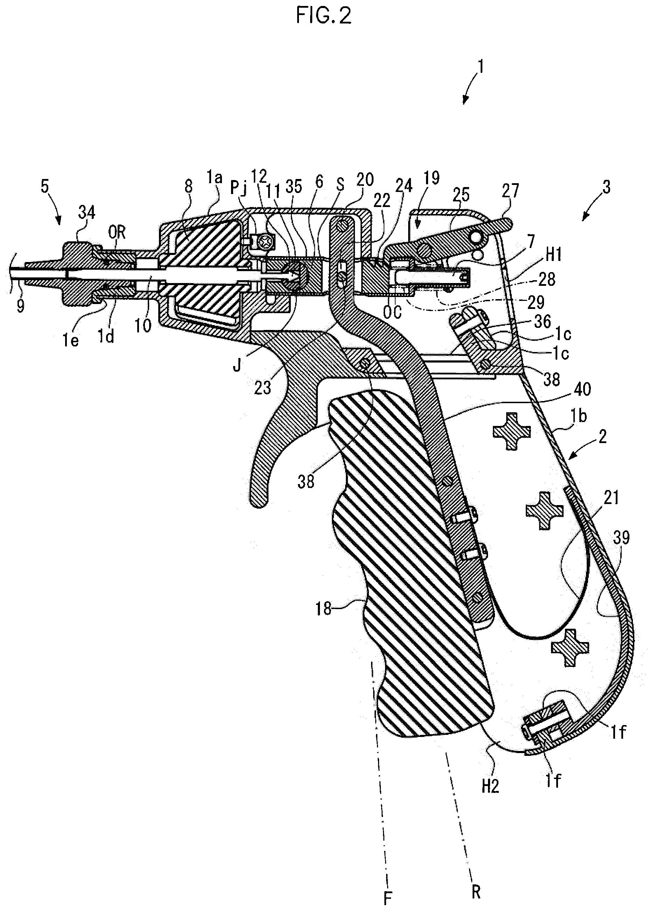

FIG. 2 illustrates a main part of the forceps device 1. As illustrated in the drawing, the forceps device 1 includes an opening/closing drive unit 6 serially coupled to a rear end portion of the opening/closing function unit 5. The opening/closing drive unit 6 is supported by the main body 3 via a sleeve S to be movable between an open position O in the front side and a closed position C in the rear side.

The opening/closing drive unit 6 is open the opening/closing portion 4 when positioned at the open position O, and closes the opening/closing portion 4 when positioned at the closed position C. The closed position C of the opening/closing drive unit 6 can be adjusted by a hexagon socket set screw 7 screwed with the main body 3 (sleeve S).

The opening/closing function unit 5 is attached to the main body 3 to be rotatable with a knob 8, and includes an outer pipe portion 9 that supports the opening/closing portion 4 to be openable and closeable at a distal end portion, a drive shaft 10 that is disposed inside the outer pipe portion 9 and driven in front-rear direction to open and close the opening/closing portion 4, and coupling means J that serially couples the drive shaft 10 to the opening/closing drive unit 6 to be releasable.

The opening/closing function unit 5 includes the shaft supporting portion 34 that supports the drive shaft 10 to be slidable in the front-rear direction. At the front end portion of the main body 3, the insertion fixing portion 1d into which the rear end side of the shaft supporting portion 34 is inserted and fixed is disposed.

The knob 8 is configured to be rotatable with a leaf spring plunger Pj, in which a leaf spring is used instead of a coil spring, while being temporarily fixed for each rotation of a predetermined angle. An O-ring OR is disposed between the shaft supporting portion 34 and the insertion fixing portion 1d of the main body 3 to seal between the main body 3 and the opening/closing function unit 5.

The coupling means J includes an enlarged-diameter portion 11 disposed at a rear end portion of the drive shaft 10 and a shaft holder 12. The enlarged-diameter portion 11 has a tapered surface with a diameter increasing toward the front side until it becomes larger than the diameter of the drive shaft 10.

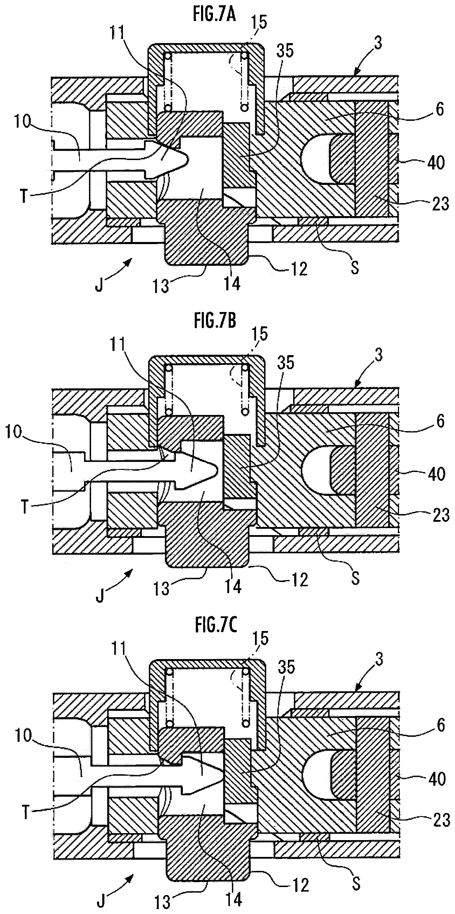

The shaft holder 12 is held by the opening/closing drive unit 6 to be movable in a left-right direction between a projection position at which a pressing portion 13 projects from the main body 3 as illustrated in FIG. 7A and a push-in position at which the pressing portion 13 is pushed into the main body 3 as illustrated in FIG. 7B, and the shaft holder 12 is biased from the push-in position in a direction of the projection position by a round wire coil spring 15 (holder biasing means).

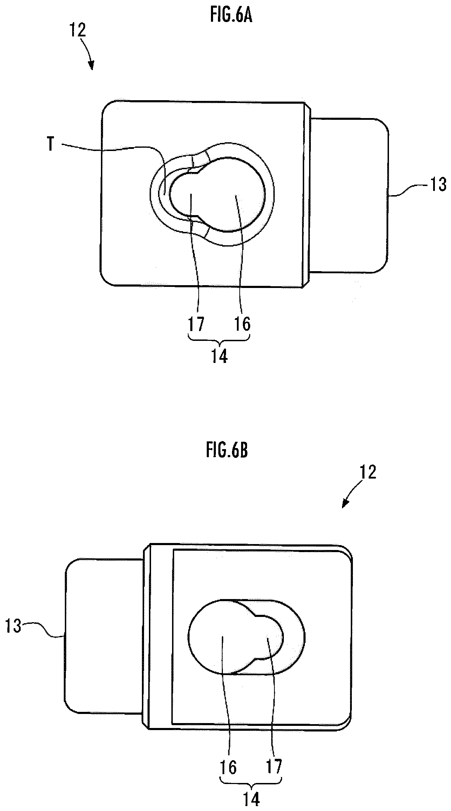

FIG. 6A illustrates a state of the shaft holder 12 viewed from the front side, and FIG. 6B illustrates a state viewed from the rear side. As illustrated in these drawings, the shaft holder 12 is provided with an engaging hole 14 for releasably capturing the enlarged-diameter portion 11 of the drive shaft 10. The engaging hole 14 includes a large-diameter portion 16 having a diameter that allows the enlarged-diameter portion 11 to pass through the large-diameter portion 16 and a U-shaped portion 17 having a width smaller than the diameter of the large-diameter portion 16. The pressing portion 13 of the shaft holder 12 projects from the main body 3 in the left direction.

The U-shaped portion 17 is positioned in the push-in position side of the shaft holder 12 with respect to the large-diameter portion 16, has the width smaller than the largest diameter of the enlarged-diameter portion 11 and larger than the diameter of the drive shaft 10, and is coupled to the large-diameter portion 16.

As illustrated in FIG. 6A, a taper-shaped portion T corresponding to the tapered shape of the enlarged-diameter portion 11 is provided in the front side of the U-shaped portion 17 of the shaft holder 12. The taper-shaped portion T contacts the tapered surface of the enlarged-diameter portion 11 to move the shaft holder 12 to the push-in position against the biasing force of the round wire coil spring 15 (holder biasing means) when the drive shaft 10 slides rearward, thereby allowing the enlarged-diameter portion 11 to pass through the large-diameter portion 16.

FIG. 7A to FIG. 7C illustrate the function of the coupling means J of the forceps device 1. When the drive shaft 10 slides rearward and becomes the states from FIG. 7A to FIG. 7C, the enlarged-diameter portion 11 of the drive shaft 10 is captured by the engaging hole 14, and the drive shaft 10 is coupled to the opening/closing drive unit 6 via the coupling means J. The captured enlarged-diameter portion 11 abuts on an abutting portion 35 provided at the opening/closing drive unit 6, and moves in accordance with the move of the opening/closing drive unit 6 between the open position O and the closed position C.

The forceps device 1 includes a movable handle 18 and a ratchet mechanism 19. The movable handle 18 is supported by the main body 3 to be movable between a forward position F spaced forward from the fixed handle 2 and a rearward position R close to the fixed handle 2 (see FIG. 2). The ratchet mechanism 19 controls the move of the opening/closing drive unit 6.

The movable handle 18 includes a turning lever 40 supported by the main body 3 to be turnable around a spindle 20 that is located above the opening/closing drive unit 6 and extends in the left-right direction. The movable handle 18 is supported by the main body 3 via the turning lever 40 to be turnable around the spindle 20 between the forward position F and the rearward position R. FIG. 2 illustrates the movable handle 18 positioned at the forward position F.

The movable handle 18 is coordinated with the opening/closing drive unit 6 such that the opening/closing drive unit 6 is positioned at the open position O when the movable handle 18 is positioned at the forward position F and the opening/closing drive unit 6 is positioned at the closed position C when the movable handle 18 is positioned at the rearward position R. This coordination is performed via an oblong hole 22 that is provided below the spindle 20 at the movable handle 18 and extends in the up-down direction and a pin-shaped member 23 that is provided to the opening/closing drive unit 6 and passes through the oblong hole 22 in the left-right direction.

That is, the movable handle 18 and the opening/closing drive unit 6 are configured such that the opening/closing drive unit 6 is driven in the front-rear direction between the open position O and the closed position C via the oblong hole 22 and the pin-shaped member 23 when the movable handle 18 is turned between the forward position F and the rearward position R.

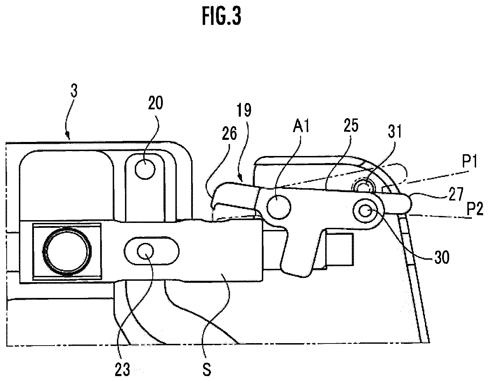

FIG. 3 illustrates the ratchet mechanism 19. The ratchet mechanism 19 transitions between a restriction state in which the move in the open position O direction of the opening/closing drive unit 6 is restricted and the move in the closed position C (see FIG. 2) direction is allowed and a restriction release state in which the move in the open position O direction and the closed position C direction is not restricted.

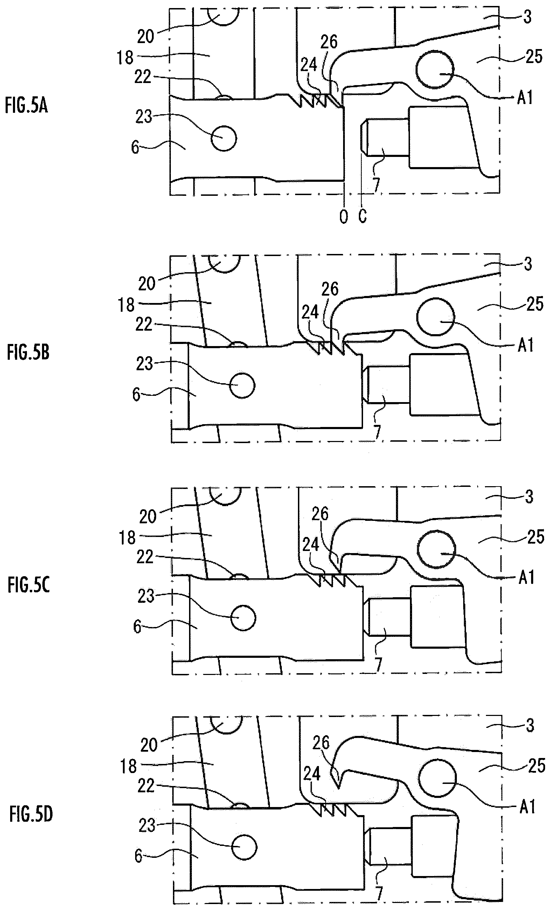

That is, the ratchet mechanism 19 includes ratchet teeth 24 (see FIG. 2, FIG. 5A) including a plurality of teeth arranged upward in the front-rear direction at the rear end portion of the opening/closing drive unit 6, and a stopper 25 coordinated with the ratchet teeth 24. The stopper 25 is supported by the main body 3 via a turning shaft A1 to be turnable between a first turning position P1 (illustrated by a two-dot chain line) at which the ratchet mechanism 19 becomes the above-described restriction state and a second turning position P2 (illustrated by a solid line) at which the ratchet mechanism 19 becomes the restriction release state. The turning shaft A1 configures a turning axis line extending in the left-right direction of the stopper 25.

The stopper 25 includes a ratchet pawl 26 collaborated with the ratchet teeth 24 and an operation portion 27 to turn the stopper 25. The ratchet pawl 26 is provided to the stopper 25 in the distal end side with respect to the turning shaft A1. The ratchet pawl 26 allows the opening/closing drive unit 6 to move rearward without meshing with the ratchet teeth 24, and restricts the move forward of the opening/closing drive unit 6 by meshing with the ratchet teeth 24 when the stopper 25 is positioned at the first turning position P1 and the ratchet mechanism 19 in the restriction state.

The operation portion 27 is provided to the stopper 25 in the rear end side with respect to the turning shaft A1 to project rearward from the main body 3, and operated to turn the stopper 25 between the first turning position P1 and the second turning position P2.

Between the main body 3 and the stopper 25, first biasing means 28 (see FIG. 2) that biases the stopper 25 from the second turning position P2 toward the first turning position P1 is provided. As the first biasing means 28, for example, a compression spring is used.

Between the main body 3 and the opening/closing drive unit 6, second biasing means 29 that biases the opening/closing drive unit 6 in a direction from the closed position C toward the open position O is provided. As the second biasing means 29, for example, a round wire coil spring can be used.

Between the main body 3 and the movable handle 18, third biasing means 21 (see FIG. 1, FIG. 2) that biases the movable handle 18 in a direction separating from the fixed handle 2 to the front side (direction from the rearward position R to the forward position F) is provided. As the third biasing means 21, a leaf spring is used in this embodiment. The second biasing means 29 and the third biasing means 21 constitute opening/closing drive unit biasing means that biases the opening/closing drive unit 6 in a direction from the closed position C toward the open position O.

Between the main body 3 and the stopper 25, fixing means that fixes the stopper 25 to the main body 3 is provided to allow the release by the operation of the operation portion 27 at the first turning position P1 and the second turning position P2.

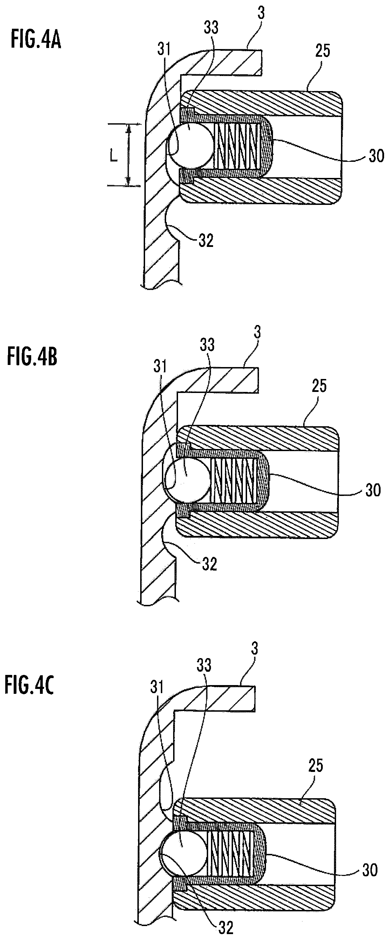

FIG. 4A to FIG. 4C illustrate the function of the fixing means. As illustrated in these drawings, the fixing means includes a ball plunger 30 provided to the stopper 25, and is provided with a first recessed portion 31 and a second recessed portion 32 formed at the right upper case 1a of the main body 3. The first recessed portion 31 and the second recessed portion 32 function as respective receiving sides of the ball plunger 30 when the stopper 25 is positioned at the first turning position P1 and the second turning position P2. The ball plunger 30 may be provided to the main body 3, and the first recessed portion 31 and the second recessed portion 32 may be provided at the stopper 25. FIG. 4A to FIG. 4C illustrate the respective states that the ball plunger 30 can have.

FIG. 5A to FIG. 5D illustrate states that the ratchet mechanism 19 can have corresponding to the respective states of the ball plunger 30. As illustrated in FIG. 4A, the first recessed portion 31 has a length L in the turning direction enough to allow the stopper 25 to slightly turn between a turning position (see FIG. 5B) at which the ratchet pawl 26 meshes with the ratchet teeth 24 and a turning position (see FIG. 5C) at which the meshing is released when the stopper 25 is positioned at the first turning position P1.

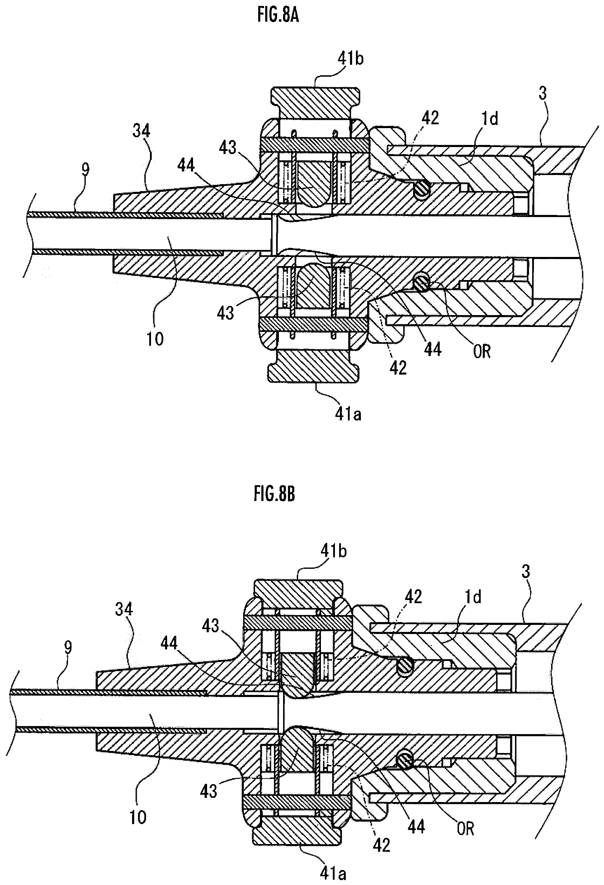

FIG. 8A and FIG. 8B illustrate an operation of the shaft supporting portion 34 when the opening/closing function unit 5 is attached to the main body 3 in the forceps device 1. As illustrated in these drawings, the shaft supporting portion 34 includes left and right press buttons 41a and 41b. Each of the press buttons 41a and 41b is supported to be movable in the left-right direction while being biased in the opposite direction of the drive shaft 10 by button biasing means 42 in the shaft supporting portion 34. As the button biasing means 42, for example, a coil spring is used.

The drive shaft 10 is provided with a contracted part having a tapered surface 44 with a diameter increasing toward the rear side. In the respective drive shaft 10 sides of the press buttons 41a and 41b, pressing portions 43 that press the tapered surface 44 to slide the drive shaft 10 rearward are provided.

Pressing the tapered surface 44 by the pressing portions 43 allows the drive shaft 10 to move in the axial direction from the position of FIG. 8A to the position of FIG. 8B. The coupling means J couples the drive shaft 10 to the opening/closing drive unit 6 based on the slide of the drive shaft 10 to the rear side.

In this configuration, when the stopper 25 of the ratchet mechanism 19 is assumed to be positioned at the second turning position P2, the ratchet mechanism 19 is in the restriction release state. FIG. 4C and FIG. 5D illustrate the state of the ball plunger 30 and the state of the stopper 25 at this situation. At this time, when it is assumed that the movable handle 18 is not applied with a force, the opening/closing drive unit 6 is positioned at the open position O by the second biasing means 29 and the third biasing means 21, and accordingly, the movable handle 18 is positioned at the forward position F.

In this state, by gripping the movable handle 18 and the fixed handle 2 with one hand and adjusting the grip strength, an operator can move the movable handle 18 to any position between the forward position F and the rearward position R against the biasing force of the second biasing means 29 and the third biasing means 21 (or with the use of the biasing force). Corresponding to this, the opening/closing drive unit 6 moves to a corresponding position between the open position O and the closed position C.

In accordance with this, the drive shaft 10 moves to the front and back, thereby opening and closing the opening/closing portion 4. Accordingly, the operator can freely open and close the opening/closing portion 4 by adjusting the grip strength with one hand.

In this state, when the operator loosens the movable handle 18 and operates the operation portion 27 of the stopper 25 with a thumb to move the stopper 25 to the first turning position P1, the ratchet mechanism 19 transitions to the restriction state. FIG. 4A and FIG. 5A illustrate the state of the ball plunger 30 and the state of the stopper 25 at this situation.

In this state, when the operation portion 27 is not applied with a force, the stopper 25 becomes the state where the ratchet pawl 26 is pressed against the ratchet teeth 24 by the first biasing means 28. At this time, as illustrated in FIG. 4A, a ball 33 of the ball plunger 30 is positioned in the upper side in the first recessed portion 31.

In this state, the opening/closing drive unit 6 is movable in the direction from the open position O toward the closed position C because the ratchet pawl 26 easily exceeds each tooth of the ratchet teeth 24. Meanwhile, in the direction from the closed position C toward the open position O, since the ratchet pawl 26 meshes with the ratchet teeth 24, the move of the opening/closing drive unit 6 is inhibited.

When the ratchet pawl 26 exceeds each tooth of the ratchet teeth 24, the position of the ball 33 of the ball plunger 30 moves from the upper side to the lower side in the first recessed portion 31 to become the state of FIG. 4B, and returns to the upper side again to become the state of FIG. 4A. Accordingly, the ratchet pawl 26 easily exceeds one tooth.

Accordingly, the operator transitions the ratchet mechanism 19 to the restriction release state to locate the opening/closing drive unit 6 at the open position O, then transitions the ratchet mechanism 19 to the restriction state (see FIG. 5A), grips the movable handle 18 and the fixed handle 2 to close the opening/closing portion 4 (see FIG. 5B), and then relaxes the grip strength. Thus, the operator can maintain the closed state of the opening/closing portion 4.

In this restriction state, by operating the operation portion 27 with the thumb to slightly turn the stopper 25 at the first turning position P1 in the direction of the second turning position P2 by a turning amount corresponding to the length L of the first recessed portion 31, the ratchet pawl 26 can be released from the meshing with the ratchet teeth 24. FIG. 4B and FIG. 5C illustrate the state of the ball plunger 30 and the state of the stopper 25 at this situation.

At this time, by decreasing the grip strength, the opening/closing drive unit 6 moves to the open position O due to the biasing forces of the second biasing means 29 and the third biasing means 21, and the opening/closing portion 4 opens. Therefore, subsequently, the operator releases the thumb from the operation portion 27 to increase the grip strength, and thus the operator can close the opening/closing portion 4 again. Then, by decreasing the grip strength, the closed state can be maintained.

Thus, even when the ratchet mechanism 19 is in the restriction state, the operator can easily open and close the opening/closing portion 4, and can easily and skillfully operate the forceps device 1 while maintaining the closed state corresponding to the situation.

When the opening/closing function unit 5 is replaced, in a state where the ratchet mechanism 19 is in the restriction release state, and therefore the movable handle 18 is positioned at the forward position F, the operator presses the pressing portion 13 of the shaft holder 12 rightward to the main body 3 against the biasing force of the round wire coil spring 15.

Thus, since the shaft holder 12 moves to the right side from the state of FIG. 7C, the enlarged-diameter portion 11 of the drive shaft 10 is released from the U-shaped portion 17 of the shaft holder 12, and aligned with the large-diameter portion 16. In this state, by holding the shaft supporting portion 34 of the opening/closing function unit 5 and pulling out the opening/closing function unit 5 from the main body 3, the enlarged-diameter portion 11 easily passes through the large-diameter portion 16, and therefore, the operator can remove the opening/closing function unit 5 from the main body 3.

Meanwhile, when the opening/closing function unit 5 is attached to the main body 3, the shaft supporting portion 34 is fitted to the insertion fixing portion 1d of the main body 3 while inserting the drive shaft 10 into the main body 3, and the enlarged-diameter portion 11 is positioned in the front side of the U-shaped portion 17 of the shaft holder 12 as illustrated in FIG. 7A. At this time, the state of the drive shaft 10 in the shaft supporting portion 34 is the state illustrated in FIG. 8A.

In this state, the operator presses the left and right press buttons 41a and 41b toward the drive shaft 10 against the biasing force of the button biasing means 42. Thus, the drive shaft 10 is pressed rearward by the pressing portion 43 via the tapered surface 44, and moves from the position of FIG. 8A to the position of FIG. 8B.

Accordingly, as illustrated in FIG. 7B, in the enlarged-diameter portion 11 of the drive shaft 10, the tapered surface contacts the taper-shaped portion T of the U-shaped portion 17 of the shaft holder 12 and presses the taper-shaped portion T, thereby moving the shaft holder 12 to the right side against the biasing force of the round wire coil spring 15. Therefore, the enlarged-diameter portion 11 passes through the large-diameter portion 16 of the engaging hole 14 and moves to the position illustrated in FIG. 7C.

Simultaneously, since the round wire coil spring 15 returns the shaft holder 12 to the original position, the enlarged-diameter portion 11 is fitted into the U-shaped portion 17 and engaged, and thus the drive shaft 10 is coupled to the opening/closing drive unit 6 as illustrated in FIG. 7C. Then, the attachment of the opening/closing function unit 5 to the main body 3 is completed.

As described above, according to the first embodiment, by making the ratchet mechanism 19 in the restriction release state, the operator can perform a procedure with one hand gripping the movable handle 18 and the fixed handle 2 while easily opening and closing the opening/closing portion 4 of the opening/closing function unit 5. Further, by making the ratchet mechanism 19 in the restriction state, the operator can cause the opening/closing portion 4 to transition from the open state to the closed state and maintain the closed state.

During the time, by operating the operation portion 27 of the stopper 25 with the thumb and moving the position of the stopper 25 between the first turning position P1 and the second turning position P2, the ratchet mechanism 19 can be easily switched between the restriction release state and the restriction state.

Additionally, the fixing means to fix the stopper 25 to the main body 3 at the first turning position P1 and the second turning position P2 is disposed between the main body 3 and the stopper 25. Therefore, the forceps device 1 can be easily operated in each of the restriction state and the restriction release state of the ratchet mechanism 19 with the thumb released from the operation portion 27 excluding a case where the operation portion 27 is slightly turned to open the opening/closing portion 4 in the restriction state (first turning position P1).

Additionally, the above-described fixing means is configured of the ball plunger 30, and the first recessed portion 31 that functions as the receiving side of the ball plunger 30 when the stopper 25 is positioned at the first turning position P1 is configured to have the above-described length L in the turning direction. Therefore, the above-described operation in the restriction state of the ratchet mechanism 19 can be easily achieved.

Additionally, the opening/closing function unit 5 is configured of the outer pipe portion 9, the drive shaft 10, the opening/closing portion 4, and the shaft supporting portion 34, and includes the coupling means J that serially couples the drive shaft 10 to the opening/closing drive unit 6 to be releasable. Therefore, the opening/closing function unit 5 can be easily replaced.

Additionally, the coupling means J couples the drive shaft 10 to the opening/closing drive unit 6 using the pressing portions 43 of the press buttons 41a, 41b pressing the tapered surface 44 of the drive shaft 10. Therefore, the drive shaft 10 can be easily coupled to the opening/closing drive unit 6 by pressing the left and right press buttons 41a, 41b after inserting the opening/closing function unit 5 into the main body 3.

Additionally, the taper-shaped portion T of the U-shaped portion 17 of the coupling means J is configured to engage the enlarged-diameter portion 11 with the U-shaped portion 17 by contacting the tapered surface of the enlarged-diameter portion 11 of the drive shaft 10 when the drive shaft 10 slides rearward and thereby moving the shaft holder 12 in the direction of being pressed into the main body 3 against the biasing force of the round wire coil spring 15. Therefore, the coupling of the drive shaft 10 to the opening/closing drive unit 6 by pressing the press buttons 41a, 41b of the shaft supporting portion 34 can be achieved by a simple configuration.

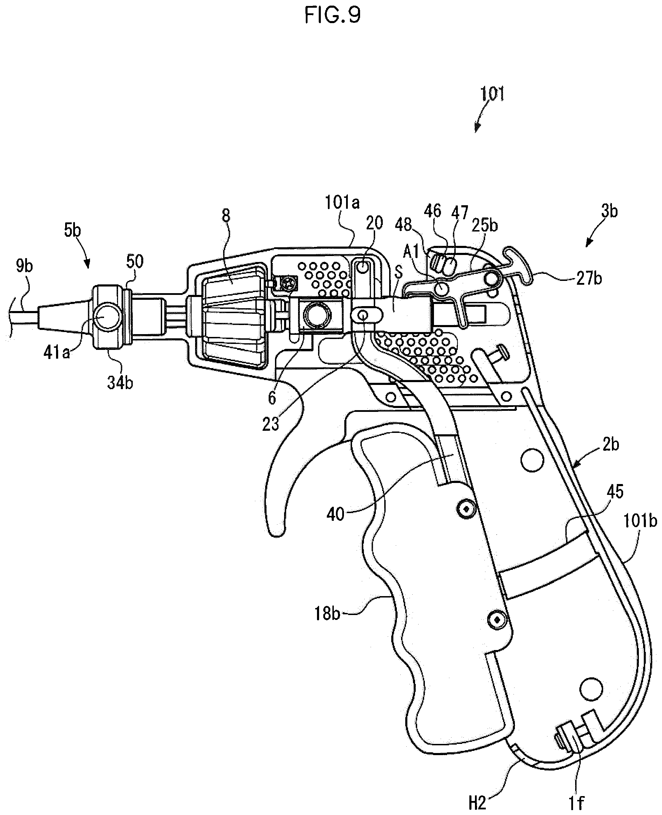

FIG. 9 illustrates a forceps device according to a second embodiment of the present invention in a state where a left case of a main body 3b is removed. In the second embodiment, components similar to those in the first embodiment are illustrated using the same reference numerals.

A forceps device 101 of FIG. 9 is different from the forceps device 1 of FIG. 1 in that, first, a movable handle 18b without an edge is employed at the movable handle, and that a compression coil spring 45 is employed instead of the leaf spring as the third biasing means that biases the movable handle 18b in the direction separating from a fixed handle 2b to the front side.

To screw right and left upper cases 101a to one another, an upright piece 46 is further provided at the inside of the right upper case 101a, and a support piece 47 supported by insides of the right and left upper cases 101a is provided. The upright piece 46 and the support piece 47 are provided above a turning shaft A1 of a stopper 25b. The right and left upper cases 101a are more strongly mutually coupled by screwing the upright piece 46 and the support piece 47 to one another.

The screwing of the upright piece 46 and the support piece 47 is performed via a void 48 above the portion in which the stopper 25b is located between the right and left upper cases 101a. The access hole H2, which is provided to screw the upright pieces 1f disposed upright on the insides of respective right and left lower cases 101b to fix the right and left lower cases 101b to one another, is narrowed compared with the first embodiment.

As the operation portion of the stopper 25b, an operation portion 27b extending along the turning direction of the stopper 25b is employed. This facilitates the operation of the stopper 25b with the thumb. Additionally, in the second embodiment, a mechanism to surely attach an opening/closing function unit 5b to the main body 3b is added.

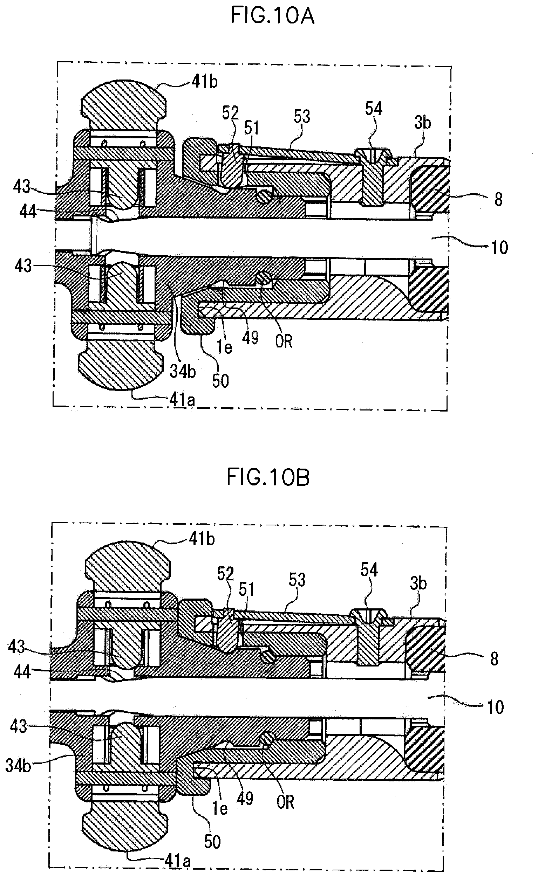

FIG. 10A and FIG. 10B are cross-sectional views illustrating a state of attaching the opening/closing function unit 5b to the main body 3b with the mechanism. As illustrated in these drawings, a shaft supporting portion 34b is provided with an engaging recess 49 at an outer surface in the rear end side. At the front end portion of the main body 3b, a through hole 51 from the outer surface of the main body 3b to the inner surface of an insertion fixing portion 50 is provided, and an engaging pin 52 is arranged inside the through hole 51.

A leaf spring 53 that biases the engaging pin 52 from the outside toward the inside of the through hole 51 is fixed to the outer surface of the main body 3b with a screw 54. The shaft supporting portion 34b is configured to be fixed to the insertion fixing portion 50 by fitting of the engaging pin 52 to the engaging recess 49 due to the biasing force of the leaf spring 53 at the insertion into the insertion fixing portion 50. The O-ring OR is provided in the rear side with respect to the engaging recess 49.

When the opening/closing function unit 5b is attached to the main body 3b, as illustrated in FIG. 10A, the shaft supporting portion 34b of the opening/closing function unit 5b is inserted inside the insertion fixing portion 50 of the main body 3b while pushing up the engaging pin 52. Further, as illustrated in FIG. 10B, the shaft supporting portion 34b is inserted until it abuts on the rear end surface of the insertion fixing portion 50.

Accordingly, the engaging pin 52 is fitted to the engaging recess 49, and the shaft supporting portion 34b is surely fixed to the insertion fixing portion 50. Simultaneously, the outside of the O-ring OR abuts on a stepped portion of the insertion fixing portion 50, and the shaft supporting portion 34b and the insertion fixing portion 50 are sealed to one another. Thus, the attachment of the shaft supporting portion 34b to the insertion fixing portion 50 is completed.

Then, similarly to the case of the first embodiment, by pressing the left and right press buttons 41a, 41b, the drive shaft 10 can be easily coupled to the opening/closing drive unit 6, and the attachment of the opening/closing function unit 5b to the main body 3b can be completed.

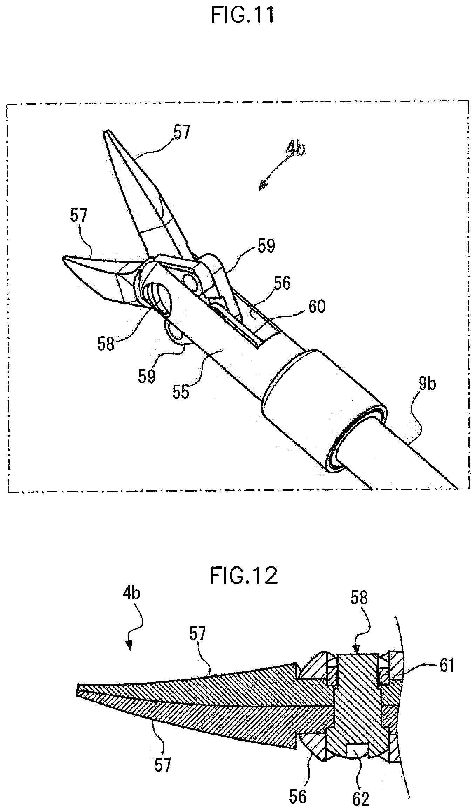

FIG. 11 illustrates a proximity of an opening/closing portion 4b of the opening/closing function unit 5b. As illustrated in FIG. 11, an outer pipe portion 9b includes an opening/closing portion holder 55 that holds the opening/closing portion 4b at the distal end. The opening/closing portion holder 55 includes a drive unit housing portion 56 having a slit shape with an open distal end side and having both sidewalls.

The opening/closing portion 4b includes two blade portions 57, a blade turning shaft 58, and two link members 59. The blade turning shaft 58 turnably couples the rear end sides of the two blade portions 57, and has both end portions supported by both sidewalls of the drive unit housing portion 56. The two link members 59 have respective distal end portions turnably coupled to the rear end portions of the blade portions 57. The two link members 59 have rear end portions coupled to one another and turnably coupled to the distal end portion of the drive shaft 10. The link members 59 are coupled to the drive shaft 10 via a cap member 60 fixed to the distal end of the drive shaft 10.

FIG. 12 illustrates a cross-sectional surface of the two blade portions 57 in the closed state taken along a surface passing through a center axis line of the blade turning shaft 58 of the two blade portions 57 and the distal ends of the respective blade portions 57. As illustrated in FIG. 12, the two blade portions 57 are provided with warpage slightly curved in the same thickness direction toward their distal ends.

That is, in the closed state, the two blade portions 57 are curved in the same direction with respect to a turning plane perpendicular to the blade turning shaft 58. Then, the opening/closing portion 4b functions as scissors having the two blade portion 57 opened and closed by driving of the drive shaft 10 in the front-rear direction.

As illustrated in FIG. 12, the blade turning shaft 58 is configured of a half thread bolt 62 fastened to the drive unit housing portion 56 with a nut 61. By adjusting the fastening force, a tissue incision performance by closing the two blade portions 57 can be optimally maintained. For other points of the second embodiment, the configurations similar to those of the first embodiment are provided.

According to the second embodiment, since the shaft supporting portion 34b is provided with the engaging recess 49, the main body 3b is provided with the through hole 51 and the engaging pin 52 at the front end portion, and the main body 3b is provided with the leaf spring 53 that biases the engaging pin 52 at the outer surface, the shaft supporting portion 34b can be surely fixed to the insertion fixing portion 50 only by inserting the shaft supporting portion 34b into the insertion fixing portion 50.

Additionally, since the opening/closing portion 4b is configured of the two blade portions 57, the blade turning shaft 58 supported by both sidewalls of the drive unit housing portion 56, and the two link members 59, the opening/closing portion 4b can be functioned as scissors.

Additionally, since the blade turning shaft 58 is configured of the half thread bolt 62 fastened with the nut 61, the tissue incision performance by the two blade portions 57 can be optimally maintained by adjusting the fastening force. Other effects of the second embodiment are similar to those of the first embodiment.

While the embodiments of the present invention have been described above, the present invention is not limited thereto. For example, while the ratchet mechanism 19 is disposed above the opening/closing drive unit 6 in each of the embodiments, the ratchet mechanism 19 may be disposed below the opening/closing drive unit 6. The opening/closing portion is not limited to one having the scissors function, and ones having various kinds of forceps functions may be employed.

### DESCRIPTION OF REFERENCE SIGNS

- 1, 101: forceps device
- 1a, 101a: upper case
- 1b, 101b: lower case
- 1c: upright piece
- 1d: insertion fixing portion
- 1e: annular groove portion
- 1f: upright piece
- 2, 2b: fixed handle
- 3, 3b: main body
- 4, 4b: opening/closing portion
- 5, 5b: opening/closing function unit
- 6: opening/closing drive unit
- 7: hexagon socket set screw
- 8: knob
- 9, 9b: outer pipe portion
- 10, 10b: drive shaft
- 11: enlarged-diameter portion
- 12: shaft holder
- 13: pressing portion
- 14: engaging hole
- 15: round wire coil spring
- 16: large-diameter portion
- 17: U-shaped portion
- 18, 18b: movable handle
- 19: ratchet mechanism
- 20: spindle
- 21: third biasing means
- 22: oblong hole
- 23: pin-shaped member
- 24: ratchet teeth
- 25, 25b: stopper
- 26: ratchet pawl
- 27, 27b: operation portion
- 28: first biasing means
- 29: second biasing means
- 30: ball plunger
- 31: first recessed portion
- 32: second recessed portion
- 33: ball
- 34, 34b: shaft supporting portion
- 35: abutting portion
- 36: fitting portion
- 37: hole
- 38: pin
- 39: reinforcement member
- 40: turning lever
- 41a, 41b: press button
- 42: button biasing means
- 43: pressing portion
- 44: tapered surface
- 45: compression coil spring
- 46: upright piece
- 47: support piece
- 48: void
- 49: engaging recess
- 50: insertion fixing portion
- 51: through hole
- 52: engaging pin
- 53: leaf spring
- 54: screw
- 55: opening/closing portion holder
- 56: drive unit housing portion
- 57: blade portion
- 58: blade turning shaft
- 59: link member
- 60: cap member
- 61: nut
- 62: bolt
- A1: turning shaft
- C: closed position
- F: forward position
- H1, H2: access hole
- J: coupling means
- O: open position
- OR: O-ring
- P1: first turning position
- P2: second turning position
- Pj: leaf spring plunger
- R: rearward position
- S: sleeve
- T: taper-shaped portion

## Claims

1. A forceps device comprising:
a main body with a fixed handle extending downward;
an opening/closing function unit having a base end portion supported by the main body and a distal end portion, the opening/closing function unit extending forward from the main body and including an opening/closing portion to be opened and closed at the distal end portion;
an opening/closing drive unit serially coupled to a rear end portion of the opening/closing function unit and supported by the main body to be movable between an open position in a front side and a closed position in a rear side, the opening/closing drive unit opening the opening/closing portion when positioned at the open position and closing the opening/closing portion when positioned at the closed position;
a movable handle supported by the main body to be movable between a forward position spaced forward from the fixed handle and a rearward position close to the fixed handle, the movable handle being coordinated with the opening/closing drive unit such that the opening/closing drive unit is positioned at the open position when the movable handle is positioned at the forward position and the opening/closing drive unit is positioned at the closed position when the movable handle is positioned at the rearward position; and
a ratchet mechanism that transitions between a restriction state in which the opening/closing drive unit is restricted to move in a direction of the open position and allowed to move in a direction of the closed position and a restriction release state in which the move in the open position direction and the closed position direction is not restricted, wherein
the ratchet mechanism includes:
ratchet teeth including a plurality of teeth arranged in a front-rear direction at a rear end portion of the opening/closing drive unit; and
a stopper supported by the main body to be turnable around a turning axis line extending in a left-right direction between a first turning position at which the ratchet mechanism is transitioned to the restriction state and a second turning position at which the ratchet mechanism is transitioned to the restriction release state,
the stopper includes:
a ratchet pawl disposed in a distal end side with respect to the turning axis line of the stopper, the ratchet pawl being configured to allow the opening/closing drive unit to move rearward without meshing with the ratchet teeth and restrict the opening/closing drive unit to move forward by meshing with the ratchet teeth when the stopper is positioned at the first turning position; and
an operation portion disposed in a rear end side with respect to the turning axis line of the stopper so as to project rearward from the main body, the operation portion being operated to turn the stopper between the first turning position and the second turning position,
first biasing means that biases the stopper from the second turning position toward the first turning position is disposed between the main body and the stopper, and
opening/closing drive unit biasing means that biases the opening/closing drive unit in a direction from the closed position toward the open position is disposed between the main body and the stopper.

2. The forceps device according to claim 1, comprising
fixing means that fixes the stopper at the first turning position or the second turning position to be releasable by the operation portion when the stopper is turned to the first turning position or the second turning position.

3. The forceps device according to claim 2, wherein
the fixing means includes:
a ball plunger disposed at one of the main body or the stopper; and
a first recessed portion and a second recessed portion formed at the other of the main body or the stopper, the first recessed portion and the second recessed portion functioning as receiving sides of the ball plunger when the stopper is positioned at the first turning position and the second turning position, and
the first recessed portion has a length in a turning direction enough to allow the stopper to slightly turn between a turning position at which the ratchet pawl meshes with the ratchet teeth and a turning position at which the meshing is released when the stopper is positioned at the first turning position.

4. The forceps device according to claim 1, wherein
the opening/closing function unit includes:
an outer pipe portion supported by the main body to be rotatable with a knob, the outer pipe portion supporting the opening/closing portion to be openable and closable at the distal end portion;
a drive shaft disposed in the outer pipe portion and driven in the front-rear direction to open and close the opening/closing portion; and
coupling means that serially couples the drive shaft to the opening/closing drive unit to be releasable.

5. The forceps device according to claim 4, wherein
the opening/closing function unit includes a shaft supporting portion that supports the drive shaft to be slidable in the front-rear direction,
the main body has a front end portion provided with an insertion fixing portion into which a rear end side of the shaft supporting portion is inserted to be fixed,
the shaft supporting portion includes left and right press buttons,
each of the left and right press buttons is supported to be movable in the left-right direction while being biased in an opposite direction of the drive shaft by button biasing means at the shaft supporting portion,
the drive shaft is provided with a contracted part having a tapered surface with a diameter increasing toward the rear side,
each of the press buttons is provided with a pressing portion in the drive shaft side, the pressing portion presses the tapered surface to slide the drive shaft to the rear side, and
the coupling means couples the drive shaft to the opening/closing drive unit based on the slide of the drive shaft to the rear side.

6. The forceps device according to claim 5, wherein
the drive shaft has a rear end portion provided with an enlarged-diameter portion having a tapered surface with a diameter increasing toward the front side until the diameter becomes larger than a diameter of the drive shaft,
the coupling means includes a shaft holder with a pressing portion,
the shaft holder is held by the opening/closing drive unit to be movable in the left-right direction between a projection position at which the pressing portion projects from the main body and a push-in position at which the pressing portion is pushed into the main body, and the shaft holder is biased in a direction from the push-in position to the projection position by the holder biasing means, and
the shaft holder is provided with an engaging hole that releasably captures the enlarged-diameter portion of the drive shaft, wherein
the engaging hole is configured of:
a large-diameter portion having a diameter that allows the enlarged-diameter portion to pass through the large-diameter portion; and
a U-shaped portion located in the push-in position side of the shaft holder with respect to the large-diameter portion, the U-shaped portion having a width smaller than a largest diameter of the enlarged-diameter portion and larger than a diameter of the drive shaft, the U-shaped portion being coupled to the large-diameter portion, wherein
a taper-shaped portion is provided in the front side of the U-shaped portion of the shaft holder, the taper-shaped portion allows the enlarged-diameter portion to pass through the large-diameter portion by contacting the tapered surface of the enlarged-diameter portion to move the shaft holder to the push-in position against the biasing force of the holder biasing means when the drive shaft slides rearward.

7. The forceps device according to claim 5, wherein
the shaft supporting portion has an outer surface in a rear end side thereof, the shaft supporting portion being provided with an engaging recess at the outer surface,
the front end portion of the main body is provided with a through hole and an engaging pin, the through hole is formed from an outer surface of the front end portion to an inner surface of the insertion fixing portion, and the engaging pin is disposed in the through hole,
the main body has an outer surface provided with a leaf spring, and the leaf spring biases the engaging pin from an outside toward an inside of the through hole, and
the shaft supporting portion is configured to be fixed to the insertion fixing portion by fitting of the engaging pin to the engaging recess due to a biasing force of the leaf spring when the shaft supporting portion is inserted into the insertion fixing portion.

8. The forceps device according to claim 1, wherein
the opening/closing drive unit biasing means includes:
second biasing means disposed between the main body and the opening/closing drive unit, the second biasing means biasing the opening/closing drive unit in a direction from the closed position toward the open position; and
third biasing means disposed between the main body and the movable handle, the third biasing means biasing the movable handle in a direction separating from the fixed handle to the frond side.

9. The forceps device according to claim 1, wherein
the movable handle is supported by the main body to be turnable around a spindle that is located above the opening/closing drive unit and extends in the left-right direction, and
the opening/closing drive unit is coordinated with the movable handle via an oblong hole that is provided below the spindle at the movable handle and extends in an up-down direction and a pin-shaped member that is provided to the opening/closing drive unit and passes through the oblong hole in the left-right direction.

10. The forceps device according to claim 4, wherein
the outer pipe portion includes an opening/closing portion holder that has a distal end and holds the opening/closing portion at the distal end,
the opening/closing portion holder includes a drive unit housing portion having a slit shape with an open distal end side and having both sidewalls, wherein
the opening/closing portion includes:
two blade portions;
a blade turning shaft that turnably couples rear end sides of the two blade portions, the blade turning shaft having both end portions being supported by both of the sidewalls of the drive unit housing portion; and
two link members having respective distal end portions turnably coupled to the rear end portions of the blade portions, the rear end portions being coupled to one another and turnably coupled to a distal end portion of the drive shaft, wherein
the two blade portions are curved in an identical direction with respect to a turning plane perpendicular to the blade turning shaft in a closed state, and function as scissors to be opened and closed by driving of the drive shaft in the front-rear direction, and
the blade turning shaft is configured of a bolt fastened to the drive unit housing portion with a nut.
